# EUROPEAN PATENT SPECIFICATION

(11) **EP 2 997 984 B1**
(45) Date of publication and mention of the grant of the patent: **03.06.2020**
(21) Application number: 15185576.4
(22) Date of filing: 17.09.2015
(51) Int. Cl.: A61L 27/18, A61L 27/34, A61L 27/54, A61L 29/06, A61L 29/08, A61L 29/16, A61L 31/06, A61L 31/10, A61L 31/16, A61L 33/00

(54) **METHOD FOR IMMOBILIZATION OF HEPARIN ON A POLYMERIC MATERIAL**
VERFAHREN ZUR IMMOBILISIERUNG EINES POLYMERMATERIALS
PROCÉDÉ POUR L'IMMOBILISATION DE L'HÉPARINE SUR UN MATÉRIAU POLYMÈRE

(30) Priority: 19.09.2014 GB 201416593
(43) Date of publication of application: 23.03.2016
(73) Proprietor: Institute Jozef Stefan, 1000 Ljubljana (SI); University of Maribor, 2000 Maribor (SI)
(72) Inventor: KOLAR, Metod, 1000 Ljubljana (SI); MOZETIC, Miran, 1000 Ljubljana (SI); JUNKAR, Ita, 1000 Ljubljana (SI); VESEL, Alenka, 1000 Ljubljana (SI); MODIC, Martina, 1000 Ljubljana (SI); KLEINSCHEK, Karin Stana, 2000 Maribor (SI)
(74) Representative: Marton, Dan-Robert

(56) References cited:
- EP-A2- 0 124 200
- WO-A1-2013/049068
- CN-A- 101 214 395
- US-A- 5 914 115
- M.Ö ÖTEYAKA ET AL: "Effect of surface modification by ammonia plasma on vascular graft: PET film and PET scaffold", ACTA PHYSICA POLONICA A, vol. 121, 1 January 2012 (2012-01-01), pages 125-127, XP055237949,

## Description

### FIELD OF INVENTION

The present invention relates to method for immobilization of heparin on polymeric material as well as materials and products produced thereby.

### BACKGROUND OF THE INVENTION

Artificial vascular grafts are widely used for treatment of obstructive atherosclerotic disease, which is a major cause of mortality in the western world, especially in cases where replacement with an autologous vascular graft is not an option. Vascular grafts have been used successfully to replace large-diameter blood vessels, however on the other hand the long term patency of small-diameter vascular grafts is still unacceptable, primarily due to stenosis and thrombus formation. Therefore, special surface modification of vascular grafts is required to overcome this problem. Recent discoveries by Cvelbar et. al. WO2009051567 A2 have successfully utilized the surface modification by means of gaseous plasma to improve biocompatibility of the prosthetic vascular grafts. However a rather large problem arises here, that is the ageing of the surface. After the surface modification, the generated functional groups start to react with the surrounding atmosphere and/or start to diffuse into the treated sample.

There have been numerous attempts to improve biocompatibility of the artificial vascular grafts, mainly by immobilizing the anticoagulant agents on the surface. The immobilized anticoagulant such as heparin improves the haemocompatibility of the artificial surface by binding to anti-thrombin III and inhibiting thrombogenesis primarily through inactivation of factors Ha and Xa. Known methods of heparin immobilization include physical adsorption, ion bonding and covalent bonding.

In WO2008039521 covalent immobilization of heparin to plasma treated polymer surface is disclosed. Surface was plasma treated either with O₂, N₂ or amine gas. NZ546981 deals with a polymer having active functional groups like amine, carboxyl and sulphhydryl groups which are capable of chemically binding biological molecules like heparin. A step of depositing biological molecules on the surface of polymer takes place in a single step by means of cold plasma methods. In KR20100059367 heparin was fixed to the polymer surface via activated carboxylic groups which were grafted to the polymer surface previously activated in plasma by means of He, Ar, N₂, O₂, H₂, CO₂ CO, NH₃ and H₂O plasma or their mixtures.

Grafting was used in CN101024150 where polyacrylic acid was grafted to the surface. Heparin was then chemically coupled by the use of 1-Ethyl-3-(3-dimethylaminopropyl)carbodiimide. Chemical modification of the polymer surface and covalent coupling of heparin by condensation of carboxyl and azyl groups in the presence of crosslinking agent was used also in CN1563157. In WO9410938 heparin was attached directly (or indirectly via spacer groups) to N-vinyl-pyrrolidone which was deposited to the polymer substrate by plasma polymerization.

In RU803193 covalent attachment was achieved by acylation of heparin and copolymerization with unsaturated polymer in the presence of cross-linking agent. In WO9314127 polyaminocations covalently immobilized with polyethylene oxide spacer to polymer surface for heparin binding is used.

In US4291133 polymer with pendant aliphatic side chains with reacting groups which may react with heparin is disclosed. In JPH06125980 heparin was ionically bonded to phosphatide polymer. In CN101966350 special heparin complex was prepared and dissolved into solution to prepare a coating liquid. EP2272549 describes immobilization of heparin by ring opening polymerization of cyclic lacton forming biodegradable co-polymer with dicarboxylic anhydride and a heparin. In CN101130902 and US2006240110 electrospinning method to prepare heparin containing fibers from mixed solution of heparin and polymer is disclosed.

In JPH0274261 preparation of antithrombogenic surface by formation of heparin treated layer due to reaction of heparin with surface reactive agent film which was prepared by using solvent with ammonium salt is disclosed. Optionally, a method can include plasma treatment of polymeric substrate before immersing into solution of ammonium salt. In CA1257561 a medical device was coated with solution containing a photosensitive polymer, heparin and cross-linking salt and exposed to UV radiation to induce crosslinking of the polymer.

Embodimens disclosed in US3617344, US3846353 US3616935 were based on binding quaternary amines to a polymer surface and subsequently ionically binding heparin thereto. These methods have the disadvantage of leaching of the antithrombogenic material rendering the system non-permanent and ineffective for long term internal use in the body.

The commercially available collagen-, gelatin-, heparin- and albumin- coated vascular grafts have overcame the above mentioned leeching problem by covalently immobilizing heparin on the surface (WO 9608149, EP 1501565, FR 2793693, DE 19724869, WO2005053765, US 4326532, US 20060228391, US 4987181 in US 20040234575).

EP 0 124 200 discloses an embodiment in which polystyrene microtiter was placed in a plasma generator and the system evacuated to 20 micron of Hg. Ammonia was bled in at such a rate that a pressure of 27 Pa was established and maintained. After a 5 minute flush at this pressure, a plasma was initiated at a frequency of 20 MHz and 40 watts power. The plasma was maintained for 5 minutes. The radio frequency was turned off and, after an additional 5 minutes flow of ammonia, the system was opened, the sample removed and heparinized. The heparinization comprised: (1) steeping in a 15% aqueous solution of dodecamethyl- methyl ammonium chloride, pH 7.5, 16 hours, 65°C, followed by thorough rinsing in distilled water and drying with nitrogen; (2) digestion in a 9% aqueous solution of sodium heparin for 16 hours at 65°C, followed by thorough rinsing with distilled water and drying with nitrogen; (3) submersion in a 1% aqueous solution of glutaraldehyde for 2 hours at 60°C, followed by thorough rinsing with distilled water, 5% aqueous Triton x 405, and distilled water.

One disadvantage of these techniques is among other things that wet methods of chemical surface treatments to produce the desired product are used.

Hence an object of the present invention is providing of an improved method for immobilization of anticoagulant substance on polymeric materials.

### SUMMARY OF THE INVENTION

The present invention is defined by the appended independent claims.

The present invention relates to a method for immobilization of heparin on a polymeric material, said method comprising the steps of:
- selecting a substrate made from a polymeric material;
- cleaning said substrate;
- mounting said substrate into a vacuum chamber;
- evacuating the vacuum chamber to pressure below 1 Pa;
- selecting a source of NH₂ radicals;
- leaking solely the NH₂ radicals from said source into said vacuum chamber during continuous pumping of said vacuum chamber;
- interacting said NH₂ radicals with said substrate;
- evacuating said vacuum chamber;
- venting said vacuum chamber to atmospheric pressure; and
- covalent bonding of activated heparin on said substrate
- wherein said cleaning step comprises:
   - treating of said substrate made from a polymeric material in ethanol or other appropriate solvent using an ultrasound bath; and
   - drying said substrate made from a polymeric material;
- characterized in that, said covalent bonding of activated heparin on said substrate is performed by interacting a water solution containing of heparin activated by N-(3-Dimethylaminopropyl)-N'-ethylcarbodiimide hydrochloride (EDC)/ N-Hydroxysuccinimide (NHS) on said substrate.

According to the invention activated heparin is bonded or coated to a pre-conditioned surface of a substrate is provided. Therefor adhesion of activated heparin on said surface of the substrate is effective showing exceptional anti-thrombogenic results.

The substrate according to the present invention may be part of an implantable medical device as stated below, thereby no coagulation of blood on the surface of the device, i.e. substrate surface, can be avoided.

According to the present invention the heparin is covalently bonded by multi-point activation. Multi-point activation is done by means of water solution containing of heparin activated by N-(3-Dimethylaminopropyl)-N'-ethylcarbodiimide hydrochloride (EDC)/N-Hydroxysuccinimide (NHS) which is then correspondingly put in contact with the substrate. By means of this method step optimal bonding of activated heparin on the surface could be reached.

Hence, it is provided a method for immobilization of heparin on a polymeric material by subsequent interacting said polymeric material with NH₂ radicals. Later on heparin can be bonded by means of water solution of heparin activated with EDC/NHS and careful drying in a limited range of temperatures. The present invention also relates to materials produces by methods of present invention and to products produced from such materials. In the preferred embodiment said polymeric material is selected from the group consisting of biopolymers, synthetic polymers, inorganic polymers, polyethylene, polypropylene, polyurethane, and polyester.

The step of selecting of NH₂ radicals according to the present invention may be performed by providing a NH source and subsequently using said NH source to provide NH₂ radicals for further processing of the inventive method. For instance plasma can be used for interacting with the NH source to provide NH₂ radicals according to the present invention. It should be noted that other processes for providing NH₂ radicals in accordance with the present invention are conceivable. The water solution containing heparin is applied on the substrate, wherein said applying step can correspond to a dipping step of said pre-treated substrate into said water solution. Subsequently heparin could be therefore immobilized on said polymeric material.

In particular the present method do not apply cationic binding of anticoagulant substance on polymeric materials, however it applies covalent binding. Such a covalent bounding is achieved according to the methods of invention in such a way that said polymeric material is essentially interacting with NH₂ radicals proceeded by covalent bonding done by single or multi-point activation of heparin.

The term "NH₂ radical" represents a molecule composed of one nitrogen and two hydrogen atoms, both hydrogen atoms being bonded to nitrogen atom with a covalent bond. Unlike ammonia molecule (NH₃) where all nitrogen valence electrons are coupled with hydrogen atoms, the NH₂ radical has one dangling bond.

Advantageously the present invention does not apply collagen as an essential interface between the polymeric material and anticoagulant substance (heparin), but applies direct covalent bonding between polymeric material and anticoagulant substance, what is realized by a modification of the polymeric material surface with NH₂ radicals.

Advantageously the present invention does not modify bulk properties of polymeric material since no treatments by UV radiation is applied. Furthermore the methods of invention do not apply surface modification of polymeric materials by gaseous plasma.

According to the present invention wet chemical method steps could be minimized to a minimum, and thereby increasing the uniformity of the treatment and decreasing the residues of reactants and/or impurities generated before, during or after the reactions.

According to one embodiment of the invention said cleaning step or pre-treating step comprises treating of said substrate made from a polymeric material in ethanol using an ultrasound bath and drying said substrate made from a polymeric material. Hence an optimal pre-treated substrate in accordance with the present invention can be provided.

According to one embodiment of the invention the method further comprises interacting said substrate with said water solution, rinsing (i.e. cleaning or washout with some suitable substances) said substrate made from a polymeric material, drying said substrate made from a polymeric material. These steps can be performed after the water solution containing heparin was applied on the substrate according to the present invention.

A particularly preferred polymeric material is poly(ethylene terephthalate) (PET).

In preferred embodiments of the invention a polymeric material is in any form including foil and fibrous material as well as fabrics either non-woven or woven or knitted.

Preferably, the processing of polymeric materials according to the methods of invention is realized at temperatures between 270 K and 310 K. Such a temperature range allows for negligible modification of polymeric materials' properties due to thermal effects.

In the preferred embodiment the polymeric materials are not exposed to any radiation source including infrared, intensive visible, ultraviolet or X-ray, gamma-radiation. A source of such radiation could be gaseous plasma, UV lamp, laser beams, electron or ion beams or any other sources. Such an absence of radiation minimizes the risk of changing structural properties of polymeric materials.

The present invention also relates to a product comprising the polymeric material of the invention. In preferred embodiments the product is selected from implantable medical products such as stents, vascular grafts, heart valves and/or catheters or the like.

### BRIEF DESCRIPTION OF THE DRAWINGS

The invention is herein described, by way of example only, with reference to the accompanying drawings, wherein:
- Fig.1: shows an arrangement for conducting a method of the invention according to one embodiment;
- Fig. 2: shows a XPS survey spectrum of a polymeric material treated according to the method of the present invention;
- Fig. 3: shows a XPS survey spectrum of untreated polymeric material and polymeric material treated with a method according to the present invention;
- Fig. 4: shows an optical image (left image was acquired at 10x magnification, the right image was acquired at 50x magnification) of untreated polymeric material incubated with fresh whole blood from a healthy donor (with reference to example 1);
- Fig. 5: shows a first optical image (left image was acquired at 10x magnification, the right image was acquired at 50x magnification) of polymeric material treated with a method according to the present invention (with reference to example 2);
- Fig. 6: shows a second optical image (left image was acquired at 10x magnification, the right image was acquired at 50x magnification) of polymeric material treated with a method according to the present invention (with reference toexample 3); and
- Fig. 7: shows a third optical image (left image was acquired at 10x magnification, the right image was acquired at 50x magnification) of polymeric material treated with a method according to the present invention (with reference to example 4).

### DETAILED DESCRIPTION OF THE INVENTION

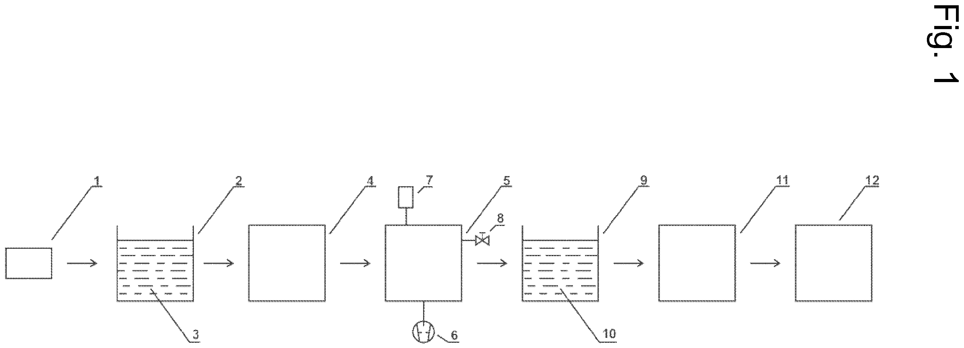

The method according to the invention encompasses a treatment of a polymeric material through a procedure shown schematically in fig. 1. The sample 1 of polymeric material is first cleaned (pre-treated) in an ultrasound bath 2 containing ethanol or similar solvent 3. This procedure allows for removal of any surface impurities.

Cleaning is followed by drying in a dry box 4, which is preferably at the room temperature. Drying is an essential step since it minimizes the risks of contamination of a vacuum chamber 5 with excessive water and/or ethanol and/or other vapor. Once the polymeric sample is dried appropriately it is mounted into the vacuum chamber 5.

The vacuum chamber 5 is evacuated using one or more vacuum pumps 6. The vacuum pumps 6 are selected in such a way that the ultimate pressure achieved in a reasonable time while keeping the vacuum system at room temperature is below 1 Pa, preferably below 0.1 Pa. Once a suitable level of vacuum inside the vacuum chamber 5 is achieved, NH₂ radicals are leaked into the vacuum chamber 5 from a source 7. Leakage is realized in such a way that the supply of NH₂ radicals or any other gasses which might be leaked intentionally or unintentionally into the vacuum chamber 5 is continuously removed by one or more pumps 6.

In a preferred embodiment the concentration of NH₂ radicals during continuous leaking and pumping is such to allow for achieving an appropriate fluence onto the polymeric material in a reasonable time, preferably between 10 s and 1000 s. The appropriate fluence according to the method of invention is between 10²² and 10²⁴ radicals/m². Such fluence allows for saturation of the surface of the polymer sample with amino functional groups. After achieving the appropriate fluence the leakage of NH₂ radicals from the source 7 is terminated, the vacuum chamber 5 is evacuated to the ultimate pressure, preferably below 1 Pa. The vacuum pumps 6 are turned off and dry air at room temperature is leaked into the vacuum chamber 5 through the vacuum valve 8. Once the pressure in the vacuum chamber 5 equals to the ambient pressure the vacuum chamber is opened and the polymeric material is immediately dipped into the water solution of heparin act with EDC/NHS 10 which has been prepared previously in an appropriate container 9.

The polymeric sample is left incubating inside the container 9 for an appropriate period of time, wherein the heparin containing water solution is applied on the substrate. In the preferred embodiment the water solution 10 in the container 9 is kept between 272 K and 282 K for several hours. Incubating stands for interacting said polymeric material with said water solution of heparin EDC for an appropriate period of time, preferably between 4 hours to 10 hours. The polymeric material is then rinsed thoroughly using an 3M solution of NaCl inside a rinsing container 11 for a period of 24 hours. After rinsing with said sodium chloride solution the container 11 is filled with deionized water in order to remove traces of NaCl and loosely or non-covalently bound heparin from the surface of polymeric material. The final step in the procedure according to the methods of invention is drying the polymeric material in dry air, preferably a desiccator 12.

The effectiveness of heparin immobilization is best determined from adsorption and activation of blood platelets on the surface of a solid material. Blood platelets adsorb in large concentrations on the surface of a solid material which exhibits high thrombogenicity. Furthermore blood platelets quickly activate on the surface of solid material which exhibits high thrombogenicity. The activation is best monitored from morphological changes of blood platelets. In inactive state the blood platelets are found in spherical shape. Upon activation on a surface of a thrombogenic material the blood platelets spread over a large surface area, so the shape is not spherical, however rather of circular flakes. The change of the blood platelets morphology is accompanied with release of certain enzymes and factors which facilitate coagulation of blood proteins and thus formation of a thrombus.

Both untreated polymeric materials as well as treated ones were incubated with fresh whole blood of a healthy donor according to the strict protocol simulating the shear force inside the blood vessel. Polymeric materials with diameter of 10 mm were incubated in 24-well cell culture microplates Blood obtained by venepuncture was collected into 4.5 ml BD Vacutainer tubes with Na-citrate as anticoagulant. 1 ml of fresh whole blood was added to the samples and incubated at 37 °C in shaker-incubator at 250 RPM. Time of incubation was 20 minutes. After incubation samples were rinsed with 1xPBS for several times in order to remove all residual blood constituents. (In order to perform further analysis, samples were fixed in 2.5 % glutaraldehyde solution at 4 °C for 25 minutes. After fixation polymers were rinsed in double deionized and air-dried.)

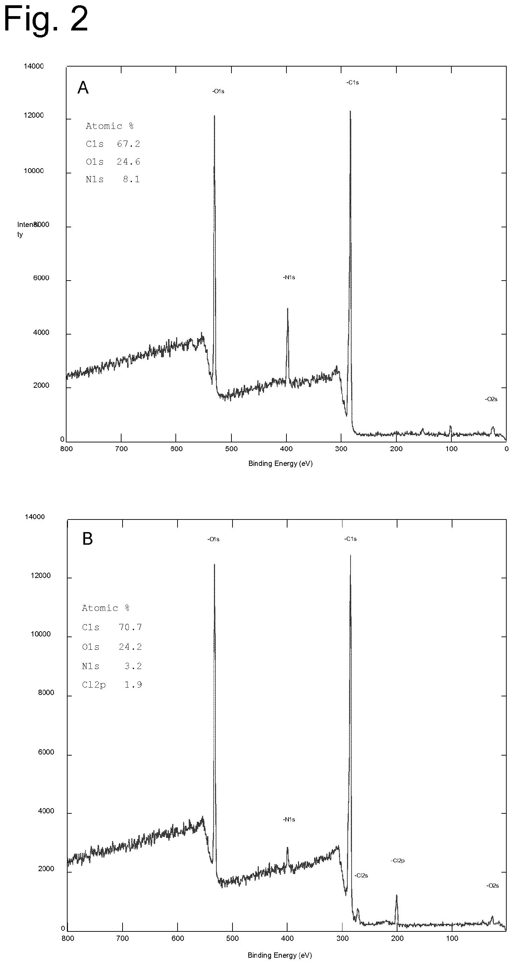

Fig. 2 shows a XPS survey spectrum of a polymeric material treated according to the method of the present invention. The polymeric material 1 is placed or mounted in a ultrasound bath 2 containing ethanol 3, dried in dry box 4 and interacted with NH₂ radicals from the source 7 inside the vacuum chamber 5 (before A and after B vapor phase derivatization with 4-chlorobenzaldehide).

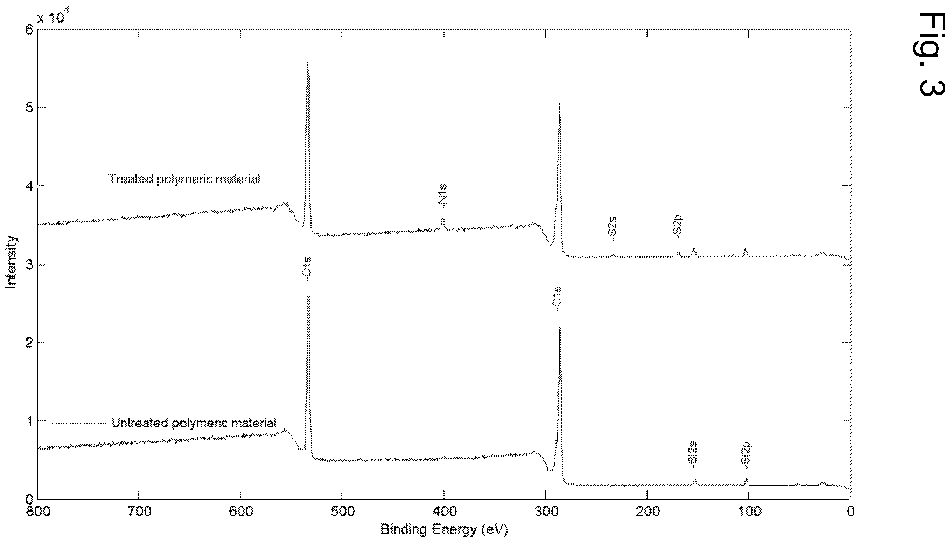

Fig. 3 shows a XPS survey spectrum of untreated polymeric material 1 and polymeric material 1 treated with a method according to the present invention.

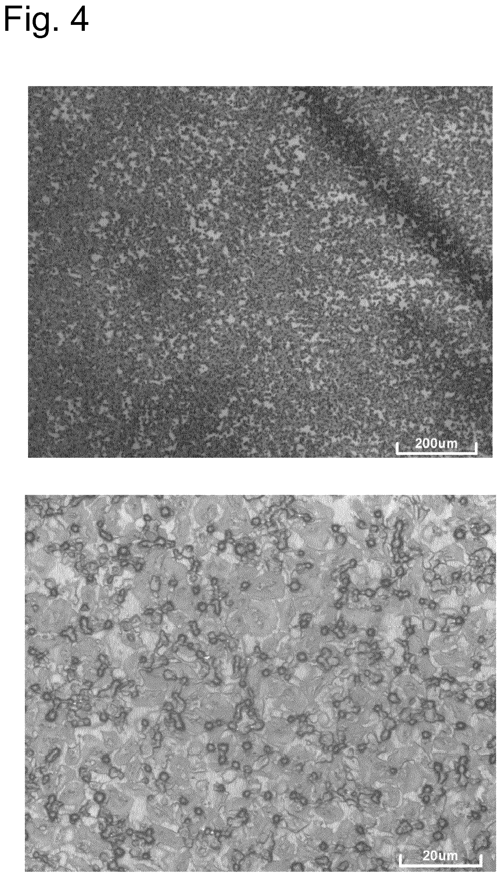

It should be noted that in figures 4 to 6 the right side shows a higher optical magnification of the substrate area shown respectively on the left side of the corresponding figure.

### EXAMPLE 1 (Comparative)

This example illustrates rather high thrombogenicity of a polymeric material. We selected poly(ethylene terephthalate) (PET) which is often applied as the material of choice in synthesizing cardiovascular implants. For this example no heparin was immobilized on the surface. The sample of this example was incubated with fresh whole blood according to the procedure described to details above. Fig. 4 shows a typical optical image of the untreated polymeric material. A great number of spread platelets observed in fig. 4 indicate thrombogenic properties of untreated PET sample used in this example. The flakes observed in this figure are highly activated blood platelets.

### EXAMPLE 2 (Comparative)

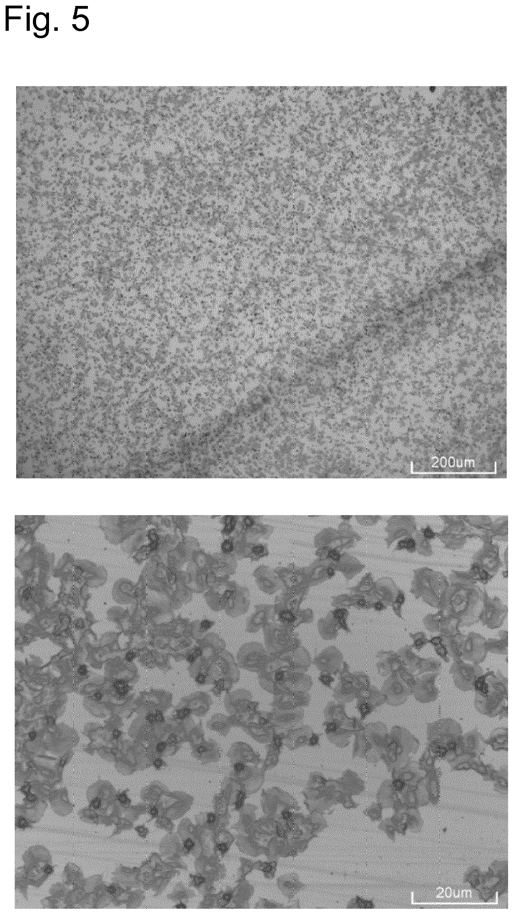

This example illustrates moderate thrombogenicity of a polymeric material. We selected poly(ethylene terephthalate) (PET) which is often applied as the material of choice in synthesizing cardiovascular implants. For this example the polymeric material was treated according to the methods of invention following all steps from 1 to 12 presented schematically in figure 1. The conditions of major step according to the methods of invention, i.e. interacting with NH₂ radicals were not optimal. The ultimate pressure in the vacuum chamber 5 during interacting with NH₂ radicals from the source 7 was 3 Pa. Such a pressure, which is higher than prescribed according to the methods of invention, does not allow for optimal immobilization of heparin on the surface of the polymeric material described in example 2. The fluence or fluency of NH₂ radicals was 1· 10²² radicals/m². The sample of this example was incubated with fresh whole blood according to the procedure described to details above. Fig. 5 shows a typical optical image of the polymeric material treated according to the methods of invention at insufficiently low pressure in the vacuum chamber 5. A small number of spread platelets observed in fig. 5 indicate improved properties of PET immobilized with heparin sample used in this example as compared to example 1. The antithrombogenic properties of this example however are still not optimal.

Fig. 5 shows an optical image of polymeric material treated according to the methods of invention following all steps from 2 to 12 as shown with reference to fig. 1 at insufficiently low pressure in the vacuum chamber 5 and incubated with fresh whole blood from a healthy donor. A small number of spread platelets observed in fig. 5 indicate improved properties of PET immobilized with heparin sample used in this example as compared to fig. 4.

### EXAMPLE 3

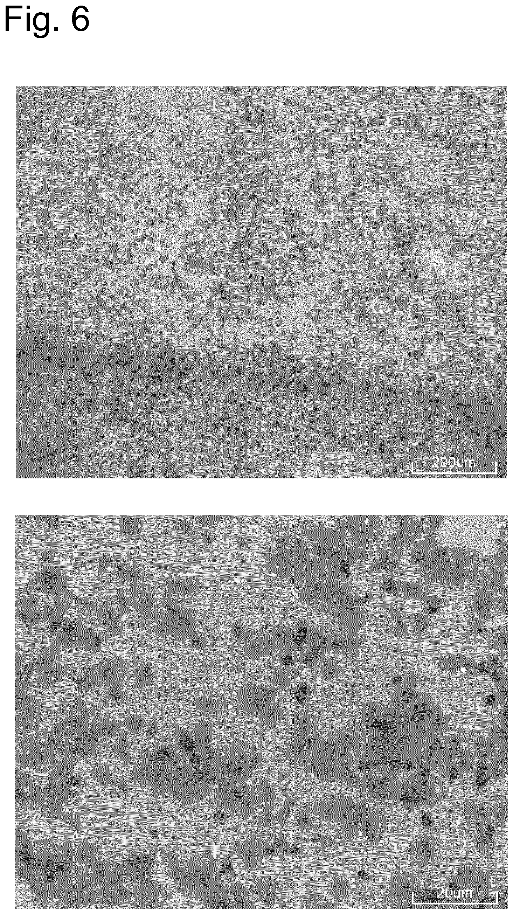

This example illustrates moderate thrombogenicity of a polymeric material. We selected poly(ethylene terephthalate) (PET) which is often applied as the material of choice in synthesizing cardiovascular implants. For this example the polymeric material was treated according to the methods of invention following all steps from 1 to 12 presented schematically in fig. 1. The conditions of major step according to the methods of invention, i.e. interacting with NH₂ radicals were not optimal. The ultimate pressure in the vacuum chamber 5 during interacting with NH₂ radicals from the source 7 was 0.1 Pa. Such a pressure is favorable according to the methods of invention. The fluence of NH₂ radicals was 1 . 10¹⁹ radicals/m², thus not an optimal value according to the methods of invention. The sample of this example was incubated with fresh whole blood according to the procedure described to details above. Fig. 6 shows a typical optical image of the polymeric material treated according to the methods of invention at insufficiently high fluence of NH₂ radical in the vacuum chamber 5. A small number of spread platelets observed in fig. 6 indicate improved properties of PET immobilized with heparin sample used in this example as compared to example 1. The antithrombogenic properties of this example however are still not optimal.

### EXAMPLE 4

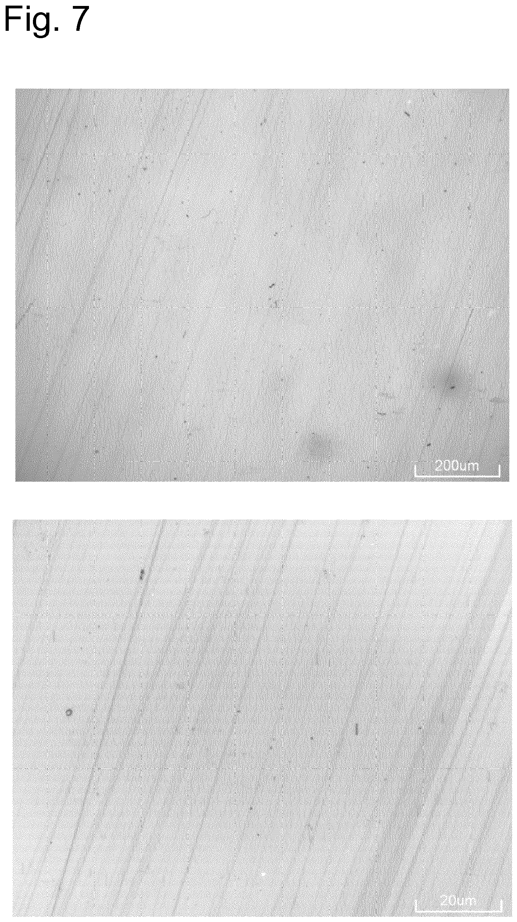

This example illustrates optimal thrombogenicity of a polymeric material. We selected poly(ethylene terephthalate) (PET) which is often applied as the material of choice in synthesizing cardiovascular implants. For this example the polymeric material was treated according to the methods of invention following all steps from 1 to 12 presented schematically in fig. 1. The conditions of all steps according to the methods of invention were optimal. The ultimate pressure in the vacuum chamber 5 during interacting with NH₂ radicals from the source 7 was 0.1 Pa. Such a pressure is favorable according to the methods of invention. The fluence of NH₂ radicals was 1 . 10²² radicals/m², thus an optimal value according to the methods of invention. The sample of this example was incubated with fresh whole blood according to the procedure described to details above. Fig. 7 shows a typical optical image of the polymeric material treated according to the methods of invention at optimal fluence of NH₂ radical in the vacuum chamber 5, evacuated to a favorite vacuum level. There is hardly any spread flake observed in fig. 7 indicating almost excellent antithrombogenic properties of PET immobilized with heparin.

The optimal antithrombogenic properties of polymeric material shown in example 4 are due to the optimal conditions during the treatment following the steps 1 to 12 according to the methods of invention. The interacting of the polymeric material with NH₂ radicals from the source 7 in the vacuum chamber 5 allows for functionalization of the surface of the polymeric material with amino groups. A confirmation of the presence of amino groups on the surface of the polymeric material after treatment in the vacuum chamber 5 is presented in fig. 2. This figure represents two XPS spectra of polymeric material. The curve A is for untreated polymeric material, and the curve B is for polymeric material treated according to the methods of invention followings steps 1 to 5. The key difference between the spectra presented in fig. 2 is appearance of chlorine on the surface of polymeric material treated according to the methods of invention following steps 1 to 5. Chlorine appears on the surface due to vapor phase derivatization with 4-chlorobenzaldehide (4-CBA). The 4-CBA is applied as a derivatizing agent who interacts solely with amino groups forming imine bond, but not with other functional groups present on the surface of untreated polymeric material. The small amount of chlorine, thus confirms the presence of amino groups on the surface of the polymeric material treated according to the methods of invention following steps 1 to 5.

Fig. 7 shows an optical image of polymeric material treated according to the methods of invention following all steps from 2 to 12 shown in fig. 1 at optimal fluence of NH₂ radical in the vacuum chamber 5, evacuated to a favorite vacuum level and incubated with fresh whole blood from a healthy donor. There is hardly any spread flake observed, indicating excellent anti-thrombogenic property of the polymeric material treating according to the methods of invention.

## Claims

1. Method for immobilization of heparin on a polymeric material, said method comprising the steps of:
- selecting a substrate made from a polymeric material;
- cleaning said substrate;
- mounting said substrate into a vacuum chamber;
- evacuating the vacuum chamber to pressure below 1 Pa;
- selecting a source of NH₂ radicals;
- leaking solely the NH2 radicals from said source into said vacuum chamber during continuous pumping of said vacuum chamber;
- interacting said NH2 radicals with said substrate;
- evacuating said vacuum chamber;
- venting said vacuum chamber to atmospheric pressure; and
- covalent bonding of activated heparin on said substrate
- wherein said cleaning step comprises:
- treating of said substrate made from a polymeric material in ethanol or other appropriate solvent using an ultrasound bath; and
- drying said substrate made from a polymeric material;
- **characterized in that,** said covalent bonding of activated heparin on said substrate is performed by interacting a water solution containing of heparin activated by N-(3-Dimethylaminopropyl)-N'-ethylcarbodiimide hydrochloride (EDC)/ N-Hydroxysuccinimide (NHS) on said substrate.

2. Method according to anyone of the preceding claims, wherein said polymeric material is selected from the group of biopolymers, synthetic polymers, inorganic polymers, polyethylene, polypropylene, polyurethane, and polyester.

3. Method according to anyone of the preceding claims, wherein said polymeric material is poly(ethylene terephthalate) (PET) in any form including foil, fiber, non-woven or woven fabrics.

4. Method of anyone of the preceding claims, wherein the partial pressures of air, nitrogen, oxygen and water vapor are below 0.1 Pa.

5. Method according to anyone of the preceding claims wherein said source of NH₂ radicals supply at least 1 . 10¹⁸ NH₂ radicals per second.

6. Method according to anyone of the preceding claims wherein the said vacuum chamber is pumping continuously during leaking of the NH₂ radicals with pumping speed at least 10 m³/h.

7. Method according to anyone of the preceding claims wherein the fluence of NH₂ radicals onto the surface of said polymeric material is between 10²⁰ and 10²⁵ radicals/m², preferably between 10²² and 10²⁴ radicals/m².

8. Method according to anyone of the preceding claims wherein said water solution containing heparin activated with N-(3-Dimethylaminopropyl)-N'-ethylcarbodiimide hydrochloride (EDC)/N-Hydroxysuccinimide (NHS) contains EDC:NHS:heparin in the molar ratio approximately 0.4:0.24:1.

9. Method according to anyone of the preceding claims wherein interacting of said substrate made from a polymeric material with said water solution lasts at least 1 hour, preferably between 5 and 10 hours.

10. Method according to anyone of the preceding claims wherein said drying of said substrate after interacting with said water solution is performed between 270 K and 310 K, preferably between 272 K and 282 K, most preferably between 274 K and 278 K.

11. A polymeric material treated by a method according to anyone of the preceding claims.

12. A product comprising a polymeric material of claim 11.

13. A product of claim 12 wherein said product is an implantable medical product, such as stents, vascular grafts, heart valves and/or catheters.

## Patentansprüche

1. Verfahren zur Immobilisierung von Heparin auf einem polymerartigen Material, wobei das Verfahren die folgenden Schritte umfasst:
- Auswählen eines Substrats, das aus einem polymerartigen Material hergestellt ist;
- Reinigen des Substrats;
- Einbringen des Substrats in eine Vakuumkammer;
- Evakuieren der Vakuumkammer auf einen Druck von weniger als 1 Pa;
- Auswählen einer Quelle von NH₂-Radikalen;
- Einleiten ausschließlich der NH₂-Radikale aus der Quelle in die Vakuumkammer, wobei die Vakuumkammer fortwährend leergepumpt wird;
- Wechselwirkenlassen der NH₂-Radikale mit dem Substrat;
- Evakuieren der Vakuumkammer;
- Belüften der Vakuumkammer bis auf Atmosphärendruck; und
- kovalentes Binden von aktiviertem Heparin an das Substrat
- wobei der Reinigungsschritt Folgendes umfasst:
- Behandeln des Substrats, welches aus einem polymerartigen Material hergestellt ist, in Ethanol oder einem anderen geeigneten Lösungsmittel unter Verwendung eines Ultraschallbads; und
- Trocknen des Substrats, welches aus einem polymerartigen Material hergestellt ist;
- **dadurch gekennzeichnet, dass** das kovalente Binden des aktivieren Heparins an das Substrat erfolgt, indem eine wässrige Lösung, die Heparin enthält, welches mittels N-(3-Dimethylaminopropyl)-N'-ethylcarbodiimid-Hydrochlorid (EDC) / N-Hydroxysuccinimid (NHS) aktiviert wurde, mit der Substratoberfläche in Wechselwirkung gebracht wird.

2. Verfahren gemäß einem beliebigen der vorhergehenden Ansprüche, wobei das polymerartige Material aus der Gruppe der Biopolymere, der synthetischen Polymere, der anorganischen Polymere, von Polyethylen, Polypropylen, Polyurethan und Polyester ausgewählt ist.

3. Verfahren gemäß einem beliebigen der vorhergehenden Ansprüche, wobei das polymerartige Material aus Polyethylenterephthalat (PET) in einer beliebigen Form, einschließlich Folien, Fasern, Vliesstoffen oder Webstoffen, ausgewählt ist.

4. Verfahren nach einem beliebigen der vorhergehenden Ansprüche, wobei die Partialdrücke von Luft, Stickstoff, Sauerstoff und Wasserdampf weniger als 0,1 Pa betragen.

5. Verfahren gemäß einem beliebigen der vorhergehenden Ansprüche, wobei die Quelle von NH₂-Radikalen mindestens 1 . 10¹⁸ NH₂-Radikale pro Sekunde bereitstellt.

6. Verfahren gemäß einem beliebigen der vorhergehenden Ansprüche, wobei die Vakuumkammer während des Einleitens der NH₂-Radikale derart fortwährend leergepumpt wird, dass die Pumpgeschwindigkeit mindestens 10 m³/Std. beträgt.

7. Verfahren gemäß einem beliebigen der vorhergehenden Ansprüche, wobei die Fluenz an NH₂-Radikalen über die Oberfläche des polymerartigen Materials zwischen 10²⁰ und 10²⁵ Radikale/m² beträgt, vorzugsweise zwischen 10²² und 10²⁴ Radikale/m².

8. Verfahren gemäß einem beliebigen der vorhergehenden Ansprüche, wobei die wässrige Lösung, welche Heparin enthält, welches mit N-(3-Dimethylaminopropyl)-N'-ethylcarbodiimid-Hydrochlorid (EDC) / N-Hydroxysuccinimid (NHS) aktiviert wurde, EDC : NHS : Heparin in einem Molverhältnis von näherungsweise 0,4 : 0,24 : 1 enthält.

9. Verfahren gemäß einem beliebigen der vorhergehenden Ansprüche, wobei die Wechselwirkung des Substrats, welches aus einem polymerartigen Material hergestellt ist, mit der wässrigen Lösung mindestens 1 Stunde, vorzugweise zwischen 5 und 10 Stunden, andauert.

10. Verfahren gemäß einem beliebigen der vorhergehenden Ansprüche, wobei das Trocknen des Substrats nach der Wechselwirkung mit der wässrigen Lösung zwischen 270 K und 310 K, vorzugsweise zwischen 272 K und 282 K, mit dem größten Vorzug zwischen 274 K und 278 K, durchgeführt wird.

11. Polymerartiges Material, das mittels eines Verfahrens gemäß einem beliebigen der vorhergehenden Ansprüche behandelt wurde.

12. Produkt, das ein polymerartiges Material nach Anspruch 11 umfasst.

13. Produkt nach Anspruch 12, wobei es sich bei dem Produkt um ein implantierbares Medizinprodukt handelt, wie etwa um Stents, Gefäßtransplantate, Herzklappen und/oder Katheter.

## Revendications

1. Procédé d'immobilisation d'héparine sur un matériau polymère, ledit procédé comprenant les étapes de :
- sélection d'un substrat fabriqué à partir d'un matériau polymère ;
- nettoyage dudit substrat ;
- montage dudit substrat à l'intérieur d'une chambre à vide ;
- évacuation de la chambre à vide jusqu'à une pression inférieure à 1 Pa ;
- sélection d'une source de radicaux NH₂ ;
- échappement uniquement des radicaux NH₂ depuis ladite source à l'intérieur de ladite chambre à vide pendant le pompage continu de ladite chambre à vide ;
- interaction desdits radicaux NH₂ avec ledit substrat ;
- évacuation de ladite chambre à vide ;
- mise à l'air de ladite chambre à vide jusqu'à la pression atmosphérique ; et
- liaison covalente d'héparine activée sur ledit substrat,
- dans lequel ladite étape de nettoyage comprend :
- le traitement dudit substrat fabriqué à partir d'un matériau polymère dans de l'éthanol ou un autre solvant approprié au moyen d'un bain à ultrasons ; et
- le séchage dudit substrat fabriqué à partir d'un matériau polymère ;
- **caractérisé en ce que** ladite liaison covalente d'héparine activée sur ledit substrat est effectuée en faisant interagir une solution aqueuse contenant de l'héparine activée par un mélange chlorhydrate de N-(3-diméthylaminopropyl)-N'-éthylcarbodiimide (EDC) / N-hydroxysuccinimide (NHS) sur ledit substrat.

2. Procédé selon la revendication précédente, dans lequel ledit matériau polymère est choisi dans le groupe constitué par les biopolymères, les polymères synthétiques, les polymères inorganiques, le polyéthylène, le polypropylène, le polyuréthane et le polyester.

3. Procédé selon l'une quelconque des revendications précédentes, dans lequel ledit matériau polymère est le poly(téréphtalate d'éthylène) (PET) sous n'importe quelle forme, y compris une feuille, une fibre, un non-tissé ou un tissu.

4. Procédé selon l'une quelconque des revendications précédentes, dans lequel les pressions partielles d'air, d'azote, d'oxygène et de vapeur d'eau sont inférieures à 0,1 Pa.

5. Procédé selon l'une quelconque des revendications précédentes dans lequel ladite source de radicaux NH₂ fournit au moins 1.10¹⁸ radicaux NH₂ par seconde.

6. Procédé selon l'une quelconque des revendications précédentes dans lequel ladite chambre à vide est pompée en continu pendant l'échappement des radicaux NH₂ avec une vitesse de pompage d'au moins 10 m³/h.

7. Procédé selon l'une quelconque des revendications précédentes dans lequel la fluence de radicaux NH₂ sur la surface dudit matériau polymère se situe entre 10²⁰ et 10²⁵ radicaux/m², de préférence entre 10²² et 10²⁴ radicaux/m².

8. Procédé selon l'une quelconque des revendications précédentes dans lequel ladite solution aqueuse contenant de l'héparine activée avec un mélange chlorhydrate de N-(3-diméthylaminopropyl)-N'-éthylcarbodiimide (EDC) / N-hydroxysuccinimide (NHS) contient EDC:NHS:héparine dans un rapport molaire d'environ 0,4:0,24:1.

9. Procédé selon l'une quelconque des revendications précédentes dans lequel l'interaction dudit substrat fabriqué à partir d'un matériau polymère avec ladite solution aqueuse dure au moins 1 heure, de préférence entre 5 et 10 heures.

10. Procédé selon l'une quelconque des revendications précédentes dans lequel ledit séchage dudit substrat après interaction avec ladite solution aqueuse est effectué entre 270 K et 310 K, de préférence entre 272 K et 282 K, idéalement entre 274 K et 278 K.

11. Matériau polymère traité par un procédé selon l'une quelconque des revendications précédentes.

12. Produit comprenant un matériau polymère de la revendication 11.

13. Produit de la revendication 12, ledit produit étant un produit médical implantable, comme des stents, des greffons vasculaires, des valves cardiaques et/ou des cathéters.
